# EUROPEAN PATENT SPECIFICATION

(11) **EP 0 462 247 B1**
(45) Date of publication and mention of the grant of the patent: **14.12.1994**
(21) Application number: 91901586.7
(22) Date of filing: 08.01.1991
(51) Int. Cl.: C07C 231/02

(54) **A PROCESS FOR PREPARING OXAMIDE**
VERFAHREN ZUR HERSTELLUNG VON OXAMID
PROCEDE DE PREPARATION D'OXAMIDE

(30) Priority: 09.01.1990 FI 900105
(43) Date of publication of application: 27.12.1991
(73) Proprietor: KEMIRA OY, SF-02271 Espoo (FI)
(72) Inventor: VUORI, Antti, Ilmari, SF-02100 Espoo (FI); MATTILA, Tapio, SF-02320 Espoo (FI)
(74) Representative: Ritter, Stephen David
(86) International application number: FI9100006
(87) International publication number: WO9110643

(56) References cited:
- CHEMICAL ABSTRACTS, vol. 87, no. 5, 1 August 1977, Columbus, Ohio, US, page 469, abstract no. 389 41s; & JP-A-7 707 916
- CHEMICAL ABSTRACTS, vol. 94, no. 21, 25 May 1981, Columbus, Ohio, US; T. OKABE et al, "Manufacturing process of oxamide", page 651, abstract no. 174 173q

## Description

The present invention relates to a process for preparing oxamide, process in which dimethyl oxalate is reacted in a methanol solution with ammonia, and the produced oxamide is separated from the solution by crystallization, and the methanol also produced in the reaction is separated from the solution by evaporation.

Oxamide is a solid, crystalline substance which is used as a slow-acting nitrogen fertilizer. Oxamide can be prepared, for example, by pyrolyzing ammonium oxalate or by allowing hydrogen cyanide to react with hydrogen peroxide.

It is also known to prepare oxamide by a reaction between dimethyl oxalate and ammonia, wherein methanol is produced in addition to oxamide. The preparation has been carried out as a batch process in a liquid phase, methanol acting as a solvent into which the dimethyl oxalate and ammonia are fed. The oxamide has been separated from the solution by crystallization, and the remaining mother liquor has been distilled for the recovery of methanol and the ammonia which has been left over from the reaction.

In the process mentioned above the aim has been in particular that no oxalic acid monomethyl ester monoamide, which is poorly soluble and appears as an intermediate product in the reaction, should be left in the final product. The said intermediate product would disturb the separation of the final product by filtration and would weaken the action of the obtained oxamide fertilizer. The solution to the problem has been to use ammonia in excess in the reaction. The process has, however, had the disadvantages of a low production capacity, as calculated per reactor volume, and difficulties of controlling the temperature of the strongly exothermal process.

The object of the present invention is to provide an improved process for preparing oxamide, avoiding the above-mentioned disadvantages of prior-art processes. The invention is characterized in that the preparation is carried out as a continuous process in which part of the ammonioxalic mother liquor from the oxamide crystallization step is directed to the evaporator for the purpose of methanol separation, and in which the evaporation residue is combined with the remainder of the mother liquor, which is recycled to the reaction step as the solvent into which the dimethyl oxalate and ammonia are fed.

One advantage gained by means of the process is that the energy-consuming complete distillation of the mother liquor is avoided, as is also its residual bottom product which would mainly be made up of uncrystallized oxamide. In the invention, the mother liquor portion separated for feeding it into the evaporator need not be evaporated dry, but part of it may be left as an easily removable liquid evaporation residue which is returned to the cycle. When methanol is evaporated continuously at the same rate as it is formed in the reaction between dimethyl oxalate and ammonia, the amount of methanol circulating in the process will remain constant.

In the process according to the invention, ammonia is needed in only a slight excess, in which case a portion, corresponding to the added excess, of the ammonia left over from the reaction leaves together with the evaporated methanol, and a portion returns to the reaction step together with the recycled mother liquor. By means of the recycling, the required amount of ammonia can be limited, and in a continuous process it is needed only in the amount of the excess which is barely sufficient to replace the small amount of ammonia which inevitably leaves the process in the evaporation.

The amount of mother liquor directed to the evaporation may be, for example, approximately 10 % of the mother liquor obtained from the crystallization step, and the evaporation can be carried out, for example, in such a manner that the amount of solution is reduced in it to approximately one-half. In this case approximately 5 % of the total amount of the mother liquor is evaporated, and the uncrystallized oxamide remains in its entirety in the process cycle.

According to one embodiment of the invention, the dimethyl oxalate is combined with the recycled mother liquor in the reaction vessel, into which gaseous ammonia is also introduced, and the reaction vessel is cooled so that the temperature of the reaction vessel will remain within the range 15-45 °C. The amount of dimethyl oxalate fed into the reaction vessel is preferably 10-20 % by weight of the amount of the mother liquor. Ammonia fed above the upper limit, 45 °C, of the temperature range would no longer substantially dissolve, and the lower limit, 15 °C, has been selected in order to keep the cooling costs at a moderate level. However, it is preferable to set at minimum 20 minutes as the average retention time of the reaction mixture in the reaction vessel.

In the process according to the invention it is preferable to use a plurality of vessels, connected in series, through which the reaction mixture is arranged to flow. This system facilitates keeping the temperature of the reaction mixture within the desired range, 15-45 °C. The first reaction vessel serving as the feeding vessel for dimethyl oxalate and ammonia may thus be followed by one or more vessels, in which the retention time of the reaction mixture may be in the order of 30 minutes or even more, and in which the oxamide produced begins to separate by crystallization.

According to an especially preferred embodiment of the invention, the reaction is carried out in two successive reaction vessels, gaseous ammonia being fed into each, and the reaction mixture is transferred from these to one or several subsequent vessels, in which the final crystallization of the oxamide takes place. By means of the division of the ammonia feed, drastic release of heat in the first vessel of the series is dampened, and thus the cooling requirement among the various vessels of the series is evened out.

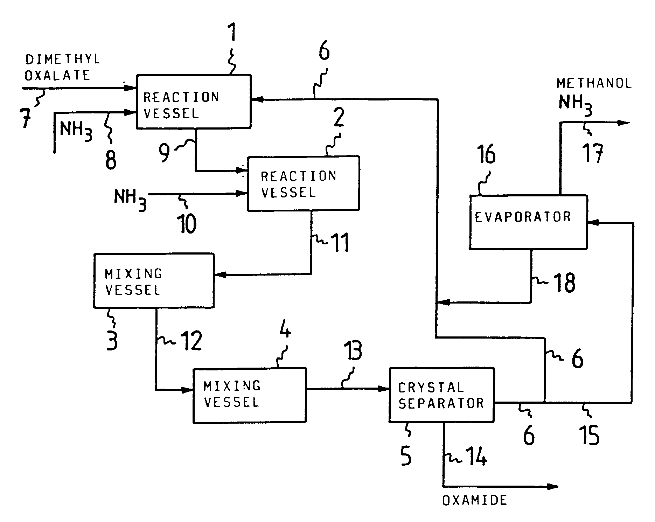

The invention is described below in greater detail, first with reference to the accompanying drawing and thereafter by illustrating the process according to the invention with embodiment examples.

The accompanying drawing is a schematic diagram of a process according to the invention, comprising five vessels 1-5 connected in series. Into a cooled reaction vessel 1 there is fed, from tube 6, recycled mother liquor, which is in the main made up of methanol but additionally contains the rather small amount of ammonia required by complete conversion and oxamide formed in the process; from tube 7, dimethyl oxalate; and from tube 8, ammonia. The reaction vessel 1 is cooled so that its temperature remains within the range 15-45 °C. The average retention time of the reaction mixture in the vessel 1 is at minimum 20 minutes, after which retention the mixture transfers via tube 9 to a second cooled reaction vessel 2, into which the balance of the ammonia required in the reaction is fed via tube 10. The temperature of the reaction mixture in the vessel 2 is maintained within the range 15-45 °C, and at minimum 30 minutes is set as the average retention time of the mixture in the vessel. From the reaction vessel 2 the mixture passes via tube 11 to a mixing vessel 3 and from there via tube 12 further to a mixing vessel 4, in which vessels the final crystallization of the oxamide which has been produced in the reactions takes place. From the mixing vessels 4 the mixture passes via tube 13 to a vessel 5, which may comprise, for example, a filter for separating crystalline oxamide from the remaining mother liquor. According to the drawing, the separated oxamide is removed via tube 14. The mother liquor, which contains methanol, ammonia which has been left over from the reaction, and uncrystallized oxamide, passes into the recycling tube 6, in which a portion of it is separated and directed into a branch tube 15, which leads to an evaporator 16. The evaporator 16 evaporates methanol and ammonia in an amount which may be, for example, approximately one-half of the solution amount separated and directed into tube 15. According to the drawing, the evaporates leave via tube 17, and the liquid evaporation residue passes via tube 18 in order to be combined with the mother liquor returning to the reaction vessel via tube 6.

In the experiments according to the following embodiment examples, the process arrangement described above was used, except that the number of reaction and mixing vessels was smaller in the experiments.

### Example 1

Dimethyl oxalate, at 47 g/h, and an ammonia-containing methanol solution which had been circulated in the process, at 423 g/h, were fed at 18 °C to a well agitated 750-ml reaction vessel. Gaseous ammonia was fed simultaneously into the said reaction vessel at a rate of 320 ml/min. The said reaction mixture was further directed at an even rate to two successive agitated 500-ml reaction vessels; in the latter vessel the product which had crystallized at 25 °C was recovered by filtration. The product obtained at an average rate of 34.6 g/h was 96.9 weight-% oxamide, which gives 98.6 % of the dimethyl oxalate as the average yield of oxamide.

### Example 2

Dimethyl oxalate, at 40 g/h, and an ammonia-containing methanol solution which had been circulated in the process, at 160 g/h, were fed at 44 °C into a well agitated 250-ml reaction vessel. Gaseous ammonia was fed simultaneously into the said reaction vessel at a rate of 140 ml/min. The obtained reaction mixture was directed at an even rate further to another well agitated 250-ml reaction vessel, into which gaseous ammonia was fed simultaneously at a rate of 150 ml/min. The reaction mixture was directed further at an even rate into an agitated 1000-ml reaction vessel, from which the product which had crystallized at 25 °C was recovered by filtration. The product obtained at an average rate of 29.5 g/h was 97.6 weight-% oxamide, which gives 97.5 % of the dimethyl oxalate as the average yield of oxamide.

### Example 3

Dimethyl oxalate, at 42.5 g/h, and filtrate of Example 2, from part of which the methanol released in the reaction had been evaporated out, at 382.5 g/h, were fed at 25 °C into a well agitated 750-ml reaction vessel. Gaseous ammonia was fed simultaneously into the said reaction vessel at a rate of 250 ml/min. The said reaction mixture was further directed at an even rate to an agitated 1000-ml reaction vessel, from which the product which had crystallized at 25 °C was recovered by filtration. The product obtained at an average rate of 31.4 g/h was 96.2 weight-% oxamide, which gives 96.3 % of the dimethyl oxalate as the average yield of oxamide.

For an expert in the art it is evident that the various embodiments of the invention are not limited to those described above as examples; they may vary within the scope of the accompanying patent claims. It is also possible that the initial substance used in the process is, instead of dimethyl oxalate, some other dialkyl ester of oxalic acid.

## Claims

1. A process for preparing oxamide, process in which dimethyl oxalate is reacted in a methanol solution with ammonia, and the formed oxamide is separated out from the solution by crystallization, and in which the methanol also produced in the reaction is separated out from the solution by evaporation, **characterized** in that the preparation is carried out as a continuous process in which part of the ammoniacal mother liquor from the crystallization step is directed to an evaporator for the separation of methanol, and in which the evaporation residue is combined with the balance of the mother liquor, which is recycled to the reaction step as the solvent into which the dimethyl oxalate and ammonia are fed.

2. A process according to Claim 1, **characterized** in that ammonia is fed into the process in a slight excess, a portion corresponding to the added excess of the ammonia left over from the reaction leaving together with the evaporated methanol and the remainder returning to the reaction step together with the recycled mother liquor.

3. A process according to Claim 1 or 2, **characterized** in that approximately 10 % of the mother liquor from the crystallization step is directed to the evaporator, and that the amount of the evaporation residue returning to the process is approximately one-half of this, approximately 5 % of the material amount of the mother liquor leaving in the evaporation.

4. A process according to any of the above claims, **characterized** in that the dimethyl oxalate is combined with the recycled mother liquor in the reaction vessel, into which gaseous ammonia is also introduced, and that the reaction vessel is cooled so that the temperature of the reaction mixture remains within the range 15-45 °C.

5. A process according to Claim 4, **characterized** in that the retention time of the reaction mixture in the reaction vessel is at minimum 20 minutes.

6. A process according to Claim 4 or 5, **characterized** in that the reaction mixture is transferred from the said reaction vessel to one or more subsequent vessels, the temperature of which is maintained within the range 15-45 °C and in which the final crystallization of oxamide takes place.

7. A process according to Claim 4 or 5, **characterized** in that the reaction takes place in two successive reaction vessels, gaseous ammonia being fed into each of them, and that the reaction mixture is transferred from these to one or more subsequent vessels, in which the final crystallization of oxamide takes place.

## Patentansprüche

1. Verfahren zum Herstellen von Oxamid, wobei Dimethyloxalat in einer Methanollösung mit Ammoniak reagiert, wobei das gebildete Oxamid aus der Lösung durch Kristallisation abgetrennt wird, und wobei das bei der Reaktion ebenfalls erzeugte Methanol aus der Lösung durch Verdampfen abgeschieden wird, dadurch gekennzeichnet, daß die Herstellung als kontinuierlicher Prozess durchgeführt wird, wobei ein Teil der in der Kristallisationsstufe angefallenen ammoniakalischen Mutterlauge einem Verdampfer zum Abscheiden des Methanols zugeleitet wird, und wobei der Verdampfungsrückstand mit der Differenzmenge der Mutterlauge kombiniert wird, die als Lösungsmittel, welchem Dimethyloxalat und Ammoniak zugegeben werden, zur Reaktionsstufe zurückgeführt wird.

2. Verfahren nach Anspruch 1, dadurch gekennzeichnet, daß das Ammoniak in geringfügigem Überschuß dem Prozess zugeführt wird, wobei ein Teil, entsprechend dem zugefuhrten Überschuß des Ammoniaks, der bei der Reaktion übriggeblieben ist, zusammen mit verdampftem Methanol austritt, und der Rest zusammen mit der rezyklierten Mutterlauge zur Reaktionsstufe zurückgeführt werden.

3. Verfahren nach Anspruch 1 oder 2, dadurch gekennzeichnet, daß annähernd 10 % der Mutterlauge aus der Kristallisationsstufe dem Verdampfer zugeführt wird, und daß die Menge des zum Prozess zurückgeführten Verdampfungsrückstandes etwa die Hälfte hiervon beträgt, annähernd 5 % der Stoffmenge der Mutterlauge, die bei der Verdampfung austritt.

4. Verfahren nach einem der vorausgegangenen Ansprüche, dadurch gekennzeichnet, daß Dimethyloxalat mit rezyklierter Mutterlauge im Reaktionsgefäß zusammengeführt wird, in welches auch gasförmiges Ammoniak eingeführt wird, und daß das Reaktionsgefäß gekühlt wird, so daß die Temperatur im Reaktionsgemisch in einem Bereich von 15-45 °C gehalten wird.

5. Verfahren nach Anspruch 4, dadurch gekennzeichnet, daß die Retentions-Zeitspanne des Reaktionsgemischs im Reaktionsgefäß wenigstens 20 Minuten beträgt.

6. Verfahren nach Anspruch 4 oder 5, dadurch gekennzeichnet, daß das Reaktionsgemisch aus dem Reaktionsgefäß in ein oder mehrere nachgeschaltete Gefäße überführt wird, in denen die Temperatur in dem Bereich von 15-45 °C gehalten wird, und in denen die abschließende Kristallisation von Oxamid stattfindet.

7. Verfahren nach Anspruch 4 oder 5, dadurch gekennzeichnet, daß die Reaktion in zwei aufeinanderfolgenden Reaktionsgefäßen stattfindet, daß in jedes dieser Gefäße gasförmiges Ammoniak eingeleitet wird, und daß das Reaktionsgemisch aus diesen in ein oder mehrere aufeinanderfolgende Gefäße überführt wird, in welchen die abschließende Kristallisation des Oxamids stattfindet.

## Revendications

1. Procédé de préparation de l'oxamide dans lequel l'oxalate de dimethyle est mis à réagir en solution méthanolique avec l'ammoniac, dans lequel l'oxamide, qui s'est formé, est séparé de la solution par cristallisation et dans lequel le méthanol qui a été également produit dans la réaction est chassé de la solution par évaporation, caractérisé en ce que la préparation est effectuée en tant que procédé en continu, dans laquelle une partie des liqueurs mères ammoniacales provenant de l'étape de cristallisation est dirigée vers un évaporateur pour l'élimination du méthanol et dans laquelle le résidu d'évaporation est combiné avec le solde des liqueurs mères, lequel est recyclé dans le stade de réaction en tant que solvant, dans lequel l'oxalate de dimethyle et l'ammoniac sont chargés.

2. Procédé selon la revendication 1 caractérisé en ce que l'ammoniac est chargé dans le procédé en léger excès, une portion correspondant à l'excès ajouté d'ammoniac restant du départ, conjointement avec le méthanol qui a été évaporé et le restant retournant à l'étape de réaction conjointement avec les liqueurs mères recyclées.

3. Procédé selon la revendication 1 ou la revendication 2 caractérisé en ce qu'environ 10 % des liqueurs mères provenant du stade de cristallisation sont dirigés vers l'évaporateur et en ce que la quantité de résidu d'évaporation retournant vers le processus est d'environ la moitié de celui-ci, approximativement 5 % de la quantité de matériau des liqueurs mères demeurant dans l'évaporation.

4. Procédé selon chacune des revendications ci-dessus caractérisé en ce que l'oxalate de diméthyle est combiné avec les liqueurs mères recyclées dans le récipient de réaction, dans lequel on introduit également de l'ammoniac gazeux et en ce que le récipient de réaction est refroidi de façon que la température du mélange réactionnel demeure dans la plage s'étendant de 15 à 45°C.

5. Procédé selon la revendication 4, caractérisé en ce que le temps de rétention du mélange réactionnel dans le récipient de réaction est au minimum de 20 minutes.

6. Procédé selon la revendication 4 ou la revendication 5 caractérisé en ce que le mélange réactionnel est transféré dudit récipient de réaction à un ou plusieurs récipients dont la température est maintenue dans la plage de 15 à 45°C, et dans lesquels a lieu la cristallisation finale de l'oxamide.

7. Procédé selon la revendication 4 ou la revendication 5 caractérisé en ce que la réaction a lieu dans deux récipients de réaction successifs, l'ammoniac étant chargé dans chacun d'entre eux et en ce que le mélange réactionnel est transféré de ceux-ci vers un ou plusieurs récipients consécutifs dans lesquels la cristallisation finale de l'oxamide a lieu.
